# EUROPEAN PATENT SPECIFICATION

(11) **EP 2 720 998 B1**
(45) Date of publication and mention of the grant of the patent: **14.12.2016**
(21) Application number: 11725644.6
(22) Date of filing: 15.06.2011
(51) Int. Cl.: C07C 67/08, C07C 69/86, B01J 23/46, C01G 55/00, B01J 37/34, B01J 35/00

(54) **METHOD FOR ESTERIFICATION AND ESTERIFICATION CATALYST**
VERFAHREN ZUR VERESTERUNG UND VERESTERUNGSKATALYSATOR
PROCÉDÉ D'ESTÉRIFICATION ET CATALYSEUR D'ESTÉRIFICATION

(43) Date of publication of application: 23.04.2014
(73) Proprietor: King Saud University, 11451 Riyadh (SA)
(72) Inventor: SIDDIQUI, Mohammed, Rafiq, Hussain, Riyadh 11451 (SA); MAHFOUZ, Refaat, Mohamed, Riyadh 11451 (SA); ALDHAYAN, Daifallah, M., Riyadh 11451 (SA); ALSHEHRI, Abdurahman, Riyadh 1145 (SA)
(74) Representative: Goddar, Heinz J.
(86) International application number: PCT/EP2011/002948
(87) International publication number: WO 2012/171539

(56) References cited:
- GB-A- 1 076 848
- US-B1- 6 278 014
- ROUSAR I ET AL: "Effect of gamma radiation on the chlorine overvoltage at a titanium anode provided with an active film of RuO2 and TiO2", ELECTROCHIMICA ACTA, ELSEVIER SCIENCE PUBLISHERS, BARKING, GB, vol. 23, no. 8, 1 August 1978 (1978-08-01) , pages 763-765, XP026516672, ISSN: 0013-4686, DOI: DOI:10.1016/0013-4686(78)80036-X [retrieved on 1978-08-01]

## Description

The present invention relates to a method for esterification.

An esterification reaction is an important reaction in the field of organic chemistry. Especially acid catalyzed esterifications are well known in the art. One famous esterification product is acetylsalicylic acid which is commonly known as aspirin. The synthesis of aspirin is a simple esterification reaction. When salicylic acid is reacted with acetic anhydride (an acid derivative), the hydroxyl group of this salicylic acid is converted into an acetyl group. The products of this esterification reaction are aspirin and acetic acid according to the following reaction scheme.

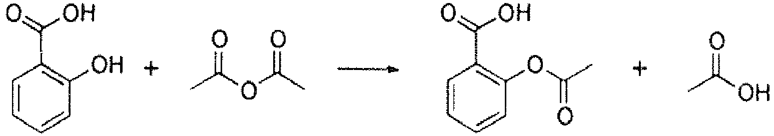

Acetic acid is considered as by product of this reaction.

Usually, for preparing aspirin small amounts of sulfuric acid (and occasionally phosphoric acid) are almost always used as a catalyst. For example US 671,769 describes process of a producing acetylsalicylic acid by substituting the acetyl group for the hydrogen of the hydroxyl group of salicylic acid and of its derivatives. The reaction is effected by the reciprocal action of salicylic acid and acetic anhydride in the presence of sulphuric acid.

Further, US 6,278,014 B1 discloses a synthesis method to obtain a reaction product containing acetylsalicylic acid and calcium and/or zinc acetate comprising heterogeneously reacting acetic anhydride, salicylic acid and at least one of calcium oxide and zinc oxide. Calcium and/or zinc oxide are not used as catalyst, but shall result in desired reaction products of calcium and/or zinc acetate.

Concentrated sulphuric acid is difficult to handle and is environmentally unfriendly.

Further, most mineral acids and phosphoric acid form polymers together with aspirin, which is, of course, a drawback for a respective preparation method.

US 6,278,014 B1 describes a method for the synthesis of acetyl salicylic acid comprising mixing acetic anhydride and salicylic acid in approximately or exactly stoichiometric proportions and calcium oxide or zinc oxide, obtaining a yield of a mixture of acetyl salicylic acid and calcium acetate or zinc acetate with 2% maximum of free salicylic acid content.

It is thus an object of the present invention to provide a method for esterification which overcomes the difficulties of the prior art. Especially a method shall be provided which is environmentally friendly utilizing a catalyst which can be easily removed from the reaction mixture and re-used.

The first object is achieved by a method for esterification of a carboxylic acid or an carboxylic acid anhydride with an alcohol in the presence of a ruthenium oxide (RuO₂) catalyst.

Preferred, the alcohol is hydroxy carboxylic acid.

More preferred, the esterification takes place according to the following chemical equation:

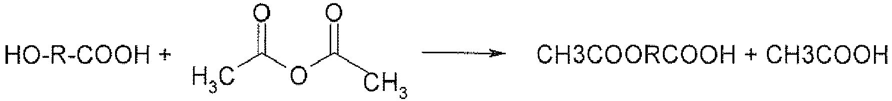

Preferably, the ruthenium oxide catalyst is calcined.

More preferably, the ruthenium oxide catalyst is a catalyst irradiated with γ-rays.

In one embodiment the ruthenium catalyst has a particle size of 1-5 nanometer.

Most preferably, the carboxylic acid anhydride is acetic anhydride and the alcohol is salicylic acid.

Further, the method is preferably carried out at a temperature of 50-100°C.

Surprisingly it was found that the method for esterification according to the invention allows esterification of carboxylic acid/anhydride and alcohol utilizing a catalyst which can be easily removed from the reaction product and re-used without significant deterioration of activity and selectivity. The method of the present invention is environmentally friendly. Especially a formation of polymers of the so far utilized acid catalysts and the preparation product can be avoided.

Any esterification reaction, preferably so far acid catalyzed esterification, can be conducted utilizing the ruthenium oxide catalyst as described in the present invention.

In a preferred embodiment, the ruthenium catalyst is irradiated prior to its use in the esterification reaction with γ-rays. This results in even improved yields.

Comparing a non-irradiated with a gamma-irradiated ruthenium oxide catalyst, it is not just the shape and size that are changed. The inventors have carried out thermal studies which really show that gamma-irradiation causes point defects and trapped electrons induced by gamma-irradiation in the host lattice, which influence the catalytic properties of the ruthenium oxide nanoparticles formed from gamma-irradiated precursor. The detailed thermal studies discussion is given below.

Additional advantages and features of the subject-matter of the present invention can be taken from the following detailed description of examples with consideration of the drawing, wherein
Fig.1 is a diagram showing thermographimetric analysis (TGA) and differential thermal analysis (DTA) curves for calcining un-irradiated Ru(acac)₃ in static air, and
Fig. 2 shows a typical diagram for TGA and DTA curves for calcining gamma-irraditated Ru(acac)₃;
Fig. 3(a) and (b) are scanning electronic microscopy measurements (SEM) of un-irradiated and gamma-irradiated RuO₂ precursor.

### 1. Preparation of ruthenium oxide catalyst

Ruthenium acetylacetonate was commercially obtained from Aldrich LTD and used without further purification. The ruthenium acetylacetonate was calcined at a temperature of about 150-700°C, preferably for about 4-36 hours. The resulting RuO₂ has a particle size of 1-5 nanometer.

A portion of the ruthenium oxide catalyst was then encapsulated under vacuum in glass vials and exposed to successively increasing doses of radiation at constant intensity using a cobalt-60 γ-ray cell 220 (Nordion, INT-INC, Ontario, Canada) at a dose rate of 10⁴Gy/h. The source was calibrated against a Fricke ferrous sulfate dosimeter and the dose rate in the irradiated sample was calculated by applying appropriate corrections on the basis of both the photon mass attenuation and energy absorption coefficient for the sample and dosimeter.

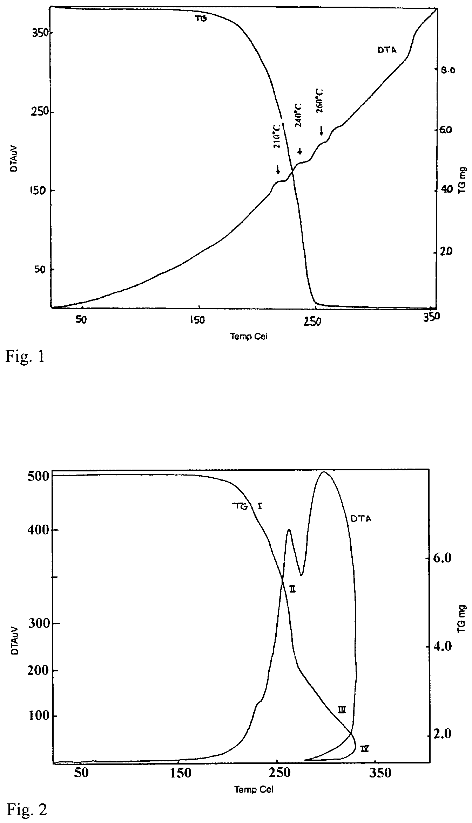

Fig. 1 shows typical TGA and TDA curves for a calcination of an-irradiated Ru(acac)₃ in static air. The TGA show that the decomposition of Ru(acac)₃ proceeds in one major step in the temperature range of 150-250°C with the formation of RuO₂ as a solid residue according to the following equation:

Ru(acac)₃ → RuO₂+volatiles

The acetylacetonate ligand was found as the major gaseous product below 300°C. At a temperature of higher than 400°C, the ligand decomposes mainly to CO, CO₂ and H₂O.

The DTA shows a superposition of several endothermic peaks in the temperature range of about 210°C to about 280°C associated with crystallization of RuO₂ and decomposition of acetylacetonate ligand.

Fig. 2 shows typical TGA and DTA curves of gamma-irradiated Ru(acac)₃ with a total dose of 10² KGy. In addition to a reduction of the induction period of the decomposition by irradiation, the TGA curve showed three overlapped decomposition stages (I-III) in the range of 150-320°C with sublimation stage IV occurred at 330°C. The three exothermic peaks centered at 230, 260 and 280°C were recorded in the DTA curve.

The significant changes in the features of the decomposition curve between un-irradiated and gamma-irradiated samples of RuO₂ could be attributed to formation of point defects and trapped electrons induced by gamma-irradiation in the host lattice.

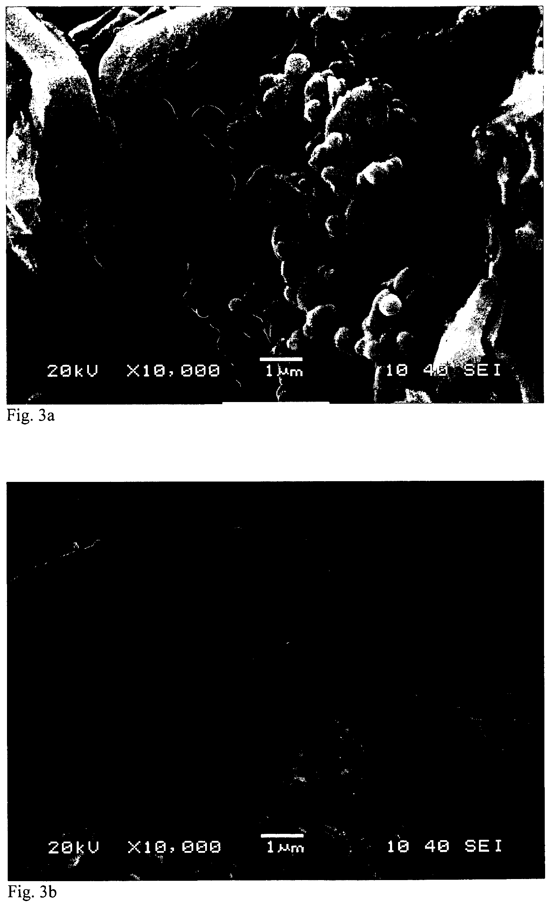

Scanning electron microscopy measurements (SEM) on the investigated nanoparticles using both un-irradiated and γ-irradiated Ru(acac)₃ precursor are shown in figures 3(a) and (b) respectively. The two images showed different morphologies, affording further evidence for the influence of γ-irradiation on shapes and sizes of as prepared nanoparticles.

### 2. Preparation of acetylsalicylic acid

To a 100 mg ruthenium oxide nanoparticles catalyst, 4 g of salicylic acid was added followed by drop-wise addition of 8 ml of dry acetic anhydride in a flask. This mixture was heated on a water bath with stirring at 60-80°C for a period of 15-20 minutes. The catalyst was then separated by filtration. After 5 minutes, the flask was placed in a beaker of ice water to accelerate crystallization and increase the yield of products. The flask was allowed to cool in the ice water for additional 5-10 minutes.

The crystals obtained were rinsed several times with chilled distilled water. Air was drawn through the filter for several minutes to accelerate drying of the crystals. The filter paper was then dried. The product was then weighed and yield calculated. The filtered catalyst was washed with water, dried in oven at 120°C and re-used.

An identical experiment was conducted, but instead of heating on a water bath, the reaction mixture was heated in microwave, maintaining the power of microwave at a lower power of 100 Watts. The mixture was heated for only 5 minutes and same work-up procedure as given above was followed.

The yield of aspirin was calculated by weighing the product and the purity tested by the following methods:
The first method used to determine the purity of aspirin was to determine the melting point. A commercial melting point apparatus was used to determine the temperature at which the crystals synthesized melt. If the aspirin is pure, it melts sharply at the literature value. If it is impure, it will be lower then the literature value by an amount that is roughly proportional to the amount of impurity present.

A more quantitative method of determining the purity of aspirin is to use absorption spectroscopy. In this method, the purified product was reacted with Fe³⁺ (aq), introduced as Fe(NO₃)₃. This will form an intensely purple-colored Fe³⁺ salicylate complex with any remaining salicylic acid impurities, but will not complex with aspirin. Absorption spectroscopy was used to measure the complex formed and to determine the amount of impurity of the aspirin obtained. All data was measured at a wavelength of 525 nm. The product was further characterized by FT-IR and NMR spectroscopy.

**Table 1 below shows the data collected for catalytic activity for the synthesis of aspirin with various catalysts.**

| Catalyst | Method | % yield |
|---|---|---|
| H₂SO₄ | Conventional | 46.3 |
| H₂SO₄ | Microwave | 53.7 |
| H₃PO₄ | Conventional | 92.6 |
| H₃PO₄ | Microwave | 94.5 |
| Ru NP_UI^{a} | Conventional | 85.2 |
| Ru_NP_I^{b} | Conventional | 88.9 |
| Ru_NP_UI^{a} | Microwave | 79.6 |
| Ru_NP_I^{b} | Microwave | 85.2 |
| Ru_NP_UI^{a} Used sec-ond run | Conventional | 83.3 |
| As above third run | Conventional | 81.5 |
| As above fourth run | Conventional | 79.6 |
| Ru_NP_I^{b} Used second run | Conventional | 81.5 |
| As above third run | Conventional | 81 |
| As above fourth run | Conventional | 79.6 |

| | | |
|---|---|---|
| Ru_NP_UI^{a} Ruthenium nanoparticles un-irradiated Ru_NP_I^{b} Ruthenium nanoparticles gamma-irradiated | | |

Respective esterifications with the amounts of salicylic acid and acetic anhydride, as given above, were also conducted using concentrated sulphuric acid and phosphoric acid as catalyst for comparative examples.

As can be taken from table 1, the esterification according to the present invention with RuO₂ catalyst results in acetylsalicylic acid in high yields which are significantly higher than the yields obtained using concentrated sulphuric as catalyst, and are somewhat comparable with the yield using phorphoric acid. In contrast to the acid catalyzed reactions the esterification method according to the present invention allows easy re-use and recycling of the catalyst. The RuO₂ catalyst can be re-used several times without significant decrease in yield (second-fourth runs).

## Claims

1. Method for esterification of a carboxylic acid or an carboxylic acid anhydride with an alcohol in the presence of a ruthenium oxide (RuO₂) catalyst.

2. Method according to claim 1, wherein the alcohol is a hydroxy carboxylic acid.

3. Method according to claim 2, wherein the esterification takes place according to the following chemical equation:

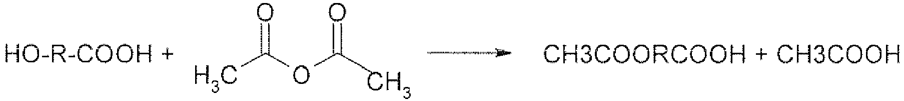

4. Method according to any of the preceding claims, wherein the ruthenium oxide catalyst is calcined.

5. Method according to any of the preceding claims, wherein the ruthenium oxide catalyst is a catalyst irradiated with gamma-rays.

6. Method according to any of the preceding claims, wherein the ruthenium oxide catalyst has a particle size of 1-5 nanometer.

7. Method according to any of the preceding claims, wherein the alcohol is salicylic acid and the anhydride is acetic anhydride.

8. Method according to any of the preceding claims, wherein the method is carried out at a temperature of 50-100°C.

## Patentansprüche

1. Verfahren zur Veresterung einer Carbonsäure oder eines Carbonsäureanhydrids mit einem Alkohol in der Gegenwart eines Rutheniumoxid-Katalysators (RuO₂).

2. Verfahren nach Anspruch 1, wobei der Alkohol eine Hydroxycarbonsäure ist.

3. Verfahren nach Anspruch 2, wobei die Veresterung gemäß der folgenden chemischen Gleichung stattfindet:

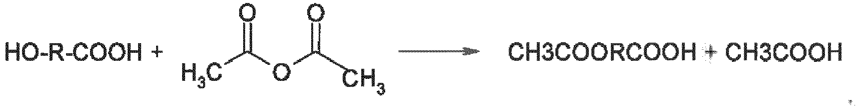

4. Verfahren nach einem der vorangehenden Ansprüche, wobei der Rutheniumoxid-Katalysator calciniert ist.

5. Verfahren nach einem der vorangehenden Ansprüche, wobei der Rutheniumoxid-Katalysator ein mit Gamma-Strahlen bestrahlter Katalysator ist.

6. Verfahren nach einem der vorangehenden Ansprüche, wobei der Rutheniumoxid-Katalysator eine Teilchengröße von 1-5 Nanometern aufweist.

7. Verfahren nach einem der vorangehenden Ansprüche, wobei der Alkohol Salicylsäure und das Anhydrid Essigsäureanhydrid ist.

8. Verfahren nach einem der vorangehenden Ansprüche, wobei das Verfahren bei einer Temperatur von 50-100° C durchgeführt wird.

## Revendications

1. Procédé d'estérification d'un acide carboxylique ou de l'anhydride d'un acide carboxylique par un alcool en la présence d'un catalyseur à oxyde de ruthénium (RuO₂).

2. Procédé selon la revendication 1, où l'alcool est un acide hydroxycarboxylique.

3. Procédé selon la revendication 2, où l'estérification a lieu selon l'équation chimique suivante :

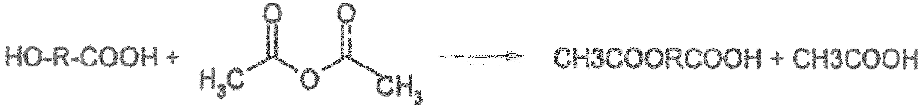

4. Procédé selon l'une quelconque des revendications précédentes, où le catalyseur à oxyde de ruthénium est calciné.

5. Procédé selon l'une quelconque des revendications précédentes, où le catalyseur à oxyde de ruthénium est irradié aux rayons gamma.

6. Procédé selon l'une quelconque des revendications précédentes, où la taille des particules du catalyseur à oxyde de ruthénium est de 1-5 nanomètres.

7. Procédé selon l'une quelconque des revendications précédentes, où l'alcool est l'acide salicylique et l'anhydride est l'anhydride acétique.

8. Procédé selon l'une quelconque des revendications précédentes, où le procédé est effectué à une température de 50-100 °C.
